# EUROPEAN PATENT SPECIFICATION

(11) **EP 1 508 613 B1**
(45) Date of publication and mention of the grant of the patent: **06.10.2010**
(21) Application number: 04018601.7
(22) Date of filing: 05.08.2004
(51) Int. Cl.: C12N 5/00, C07K 14/78

(54) **Modified reconstituted basement membrane composition for assay systems**
Zusammensetzung einer modifizierten rekonstituierten Basalmembran für Testsysteme
Composition d'une membrane basale reconstituée modifiée pour des systèmes de teste

(30) Priority: 20.08.2003 US 496413 P; 02.08.2004 US 909636
(43) Date of publication of application: 23.02.2005
(73) Proprietor: Becton, Dickinson and Company, Franklin Lakes, New Jersey 07417 (US)
(72) Inventor: Wu, Min, Carlisle MA 01741 (US); Mannuzza, Frank J., Chelmsford MA 01824 (US)
(74) Representative: von Kreisler Selting Werner

(56) References cited:
- US-A- 5 643 787
- KUBOTA Y ET AL: "Role of laminin and basement membrane in the morphological differentiation of human endothelial cells into capillary-like structures." THE JOURNAL OF CELL BIOLOGY. OCT 1988, vol. 107, no. 4, October 1988 (1988-10), pages 1589-1598, XP002327968 ISSN: 0021-9525
- NICOSIA R F ET AL: "Fibronectin promotes the elongation of microvessels during angiogenesis in vitro." JOURNAL OF CELLULAR PHYSIOLOGY. MAR 1993, vol. 154, no. 3, March 1993 (1993-03), pages 654-661, XP002327969 ISSN: 0021-9541
- PASSANITI A ET AL: "A simple, quantitative method for assessing angiogenesis and antiangiogenic agents using reconstituted basement membrane, heparin, and fibroblast growth factor." LABORATORY INVESTIGATION; A JOURNAL OF TECHNICAL METHODS AND PATHOLOGY. OCT 1992, vol. 67, no. 4, October 1992 (1992-10), pages 519-528, XP009047492 ISSN: 0023-6837
- BD BIOSCIENCES: "Product Specification Sheet - BD Matrigel TM Basement Membrane Mix"[Online] 15 February 2002 (2002-02-15), pages 1-3, XP002328319 Retrieved from the Internet: URL:http://www.bdbiosciences.com/discovery _labware/Products/pdf/matrigel_webspec.pdf > [retrieved on 2005-05-17]

## Description

### Field of Invention

The present invention relates generally to an extracellular matrix composition and cell culturing system to allow assessment of the effects of potential stimulators or inhibitors on various cell cultures.

### Background of the Invention

The harvest of cells from tissue for maintenance and propagation *in vitro* by tissue culture is a major tool in medical and biochemical research. Tissue culture is the technique or process of proliferating and/or supporting the metabolism of tissues or cells derived from organisms (plant or animal) in a formulated nutritive environment. Once isolated by gentle tissue dissociation, cells are incubated in nutritive media capable of supporting life functions. With few exceptions, cells require attachment to a substratum in order to perform normal metabolic functions, grow and divide. In tissue, the substratum, which provides the support for cell growth, is either the basement membrane or interstitial matrix. Basement membranes act not only as physical scaffolds and molecular filters, but also as solid-phase regulators of a variety of cellular processes, including attachment, motility and differentiation. Basement membranes can also modulate cellular growth by acting as a reservoir for growth factors and by prolonging their *in vivo* half-life.

The basement membrane is a specific type of extracellular matrix and is composed primarily of laminin and type IV collagen. The four major matrix components characteristic of basement matrices include laminin, collagen IV, entactin, nidogen and heparan sulfate proteoglycans (HSPG). The basement membrane also contains a number of growth factors, such as EGF, IGF-1, PDGF, TGF-*beta*, VEGF, and bFGF. Examples of commercially available basement membrane-derived extracellular matrices include, for example, ECM gel by Sigma-Aldrich, or Matrigel® by Becton, Dickinson & Company, which is extracted from the Englelbreth-Holm-Swarm (EHS) mouse tumor. This mouse tumor is rich in basement membrane proteins. At room temperature, Matrigel® Matrix gels to form reconstituted basement membrane and is similar in its structure, composition, physical property and ability to retain functional characteristics typical of basement membranes *in vivo.*

Matrigel® matrix supports differentiation of many cell types, including endothelial cells, epithelial cells, neurons and hepatocytes. When endothelial cells are plated on Matrigel®, the cells stop proliferating, display high motility and cell-cell communication. Furthermore, the cells align and form a three-dimensional network of tube or duct-like structures. The formation of these structures has been used as a model of endothelial cell differentiation, as well as the final step of the angiogenic cascade.

The use of Matrigel® in endothelial cell tube formation assays is one of the major *in vitro* applications of Matrigel® matrix. In these assays, the extent of endothelial cell tube length is measured and used as an indicator of endothelial cell tube formation. On typical extracellular matrix cell differentiation, cell tube formation can occur even in the absence of exogenously added growth factors due to the presence of growth factors in the matrix. This has limited the application of the extracellular matrix to studies involving inhibitory effects. In particular, it has been difficult to study stimulatory effects because the background tube formation is too high. As a result, tube formation assays have thus far not been sensitive enough for stimulation studies.

It is thus, one object of the present invention to provide a modified reconstituted basement membrane-derived extracellular matrix composition to reduce background cell tube formation, in the absence of added growth factors, by altering the matrix structure and/or by sequestering or blocking accessibility of the growth factors in the composition to cells.

Another object of the present invention is to provide a cell culturing system that allows direct assessment of potential angiogenesis stimulators, as well as inhibitors for their effects on cell tube formation of endothelial cells, and other cell types. It would also be beneficial to provide an assay system for studying action pathways of these stimulatory and inhibitory agents.

Additionally, an object of the present invention is to provide an assay system which is rapid and cost-effective, and which significantly increases the signal dynamic range.

### Summary of the Invention

The present invention relates to a modified reconstituted extracellular matrix composition and cell culturing system to allow assessment of potential effects of stimulators and/or inhibitors on various cell cultures. In some embodiments, the extracellular matrix composition is a modified reconstituted basement membrane composition. The inventive composition includes an extracellular matrix; and at least one exogenous component selected from: heparin, fibronectin and laminin. The pH of the composition can be adjusted to a basic pH. The extracellular matrix compositions provided herein are useful for coating a surface, such as a surface of a cell culture substrate. The coated substrates can be employed in, for example, endothelial cell tube formation assay systems.

In some embodiments, the present invention provides an extracellular matrix composition including an extracellular matrix; and exogenous fibronectin and heparin, the composition having a basic pH, wherein the extracellular matrix has a concentration of about 10 mg/ml, the exogenous fibronectin is present in concentrations of about 5 mg/ml to about 20 mg/ml and exogenous heparin is present in concentrations of about 200 mg/ml to about 800 mg/ml.

Moreover, in some embodiments, the present invention provides an extracellular matrix composition including an extracellular matrix; and exogenous laminin, the composition having a basic pH, wherein the extracellular matrix has a concentration of about 10 mg/ml and the exogenous laminin is present in concentrations of about 1 mg/ml to about 10 mg/ml.

Another aspect of the present invention is directed to a cell culturing system, which employs the inventive compositions provided herein as coating compositions for a substrate. The system includes a substrate; and a coating thereon of a coating composition including an extracellular matrix; and at least one exogenous component selected from heparin, fibronectin and laminin. The substrate may be a cell-culture substrate formed of any number of polymeric materials, glass, metal and microporous filters, for example.

Furthermore, the present invention provides a method of preparing the inventive compositions. The method includes: providing an extracellular matrix; and combining the provided extracellular matrix with at least one exogenous component selected from heparin, fibronectin and laminin. The method may further include adjusting the pH of the composition to a basic pH and/or adding sodium chloride to the composition.

Another aspect of the present invention is directed to a method for preparing the inventive cell culturing system. This method includes providing an extracellular matrix composition including an extracellular matrix and at least one exogenous component selected from heparin, fibronectin and laminin; and applying the composition to a substrate. The method further includes incubating the applied composition to allow polymerization thereof.

The compositions and systems of the present invention allow for evaluation of the ability of potential stimulators, as well as inhibitors to affect cell tube formation. Moreover, the inventive compositions significantly increase the signal dynamic range in cell tube formation assay systems.

### Brief Description of the Drawings

FIG. 1 is a schematic representation of an endothelial cell formation assay in a 96-well format, wherein the wells are coated with an extracellular matrix composition of the present invention (modified matrix).

FIG. 2 is a graph showing the effect of different concentrations of stimulatory agents on cell tube formation on a modified extracellular matrix of the present invention versus a standard extracellular matrix.

FIG. 3A is a graph showing the dose response of bFGF in inducing HUVEC tube formation on a modified extracellular matrix of the present invention.

FIG. 3B is a graph showing the dose response of sphinosine-1-phosphate (S1P) in inducing HUVEC tube formation on a modified extracellular matrix of the present invention.

FIG. 4 is a graph showing the dose response of Suramin in HUVEC tube formation induced by stimulators, bFGF and S1P, on a modified extracellular matrix of the present invention.

FIG. 5 is a graph showing the effect of inhibitors, SU5402 and Suramin, on HUVEC tube formation induced by stimulators, bFGF and S1P, on a modified extracellular matrix of the present invention.

### Detailed Description of the Invention

A rapid and versatile endothelial cell tube formation assay system is highly desirable in angiogenesis research and drug discovery. Current assay systems are effective for studying angiogenesis inhibitors, but are not effective for the study of stimulators. The present invention is directed to a modified reconstituted extracellular matrix composition, which when used in an assay system is effective for studying both angiogenesis activation and inhibition. When employed in a cell based assay system, the inventive compositions were discovered to significantly increase the signal dynamic range, and enhance direct assessment of endothelial cell capillary-like structure formations. The present invention provides a medium for direct assessment of cell tube formation stimulators and inhibitors on various coated substrates.

In some embodiments, the present invention is directed to a modified reconstituted basement membrane, which includes components that alter the structure of the solidified gel and/or alter the accessibility of growth factors within the composition to cells such that cell tube formation (cell differentiation) is minimal in the absence of exogenous stimulators.

The present invention provides an extracellular matrix composition, which includes an extracellular matrix and at least one exogenous component selected from heparin, fibronectin and laminin. In one embodiment, the extracellular matrix is a basement membrane protein extract.

In some embodiments, the inventive composition has a basic pH. The pH of the composition is preferably between about 7.8 to about 8.5, more preferably about 8.0. The pH may be maintained with a Tris (hydroxymethyl) amino methane buffer. However, it is well within the contemplation of the present invention that any buffering component capable of maintaining the composition within the pH range of about 7.8 to 8.5 is suitable. Potential buffer systems for pH 8.0 include, but are not limited to, diethanolamine, triethanolamine, (1,3-bis(tris[Hydroxymethyl]methylamino)propane); 3-[N,N-bis(2-Hydroxyethyl)amino]-2-hydroxypropanesulfonic acid: DIPSO; (N-[2-Hydroxyethyl]piperazine-N'-[-4-butanesulfonic acid] HEPBS); (N-(4-(2-hydroxyethyl-1-piperazineethanesulfonic acid: HEPES); 3-(N-Morpholino)butane sulfonic acid: MOBS); (Piperazine-N,N'-bis[2-hydroxypropanesulfonic acid: POPSO); (N-tris(Hydroxymethyl)methyl-3-aminopropanesulfonic acid: TAPS; 3-(N-tris[Hydroxymethyl]methylamino)-2-hydroxypropanesulfonic acid: TAPSO); (N-tris(Hydroxymethyl)methyl-2-aminoethanesulfonic acid: TES; (N-tris(Hydroxymethyl)methylglycine: Tricine; N-ethylmorpholine, dimethylleucylglycine, sodium 5:5-diethyl barbituate and 2 amino, 2 methyl-1:3 propanediol.

In some embodiments, the inventive composition can include a Tris buffer at concentrations sufficient to buffer in the basic pH range. When present, the Tris buffer is preferably present at a concentration of about 0.01 M to about 0.05 M of the total composition. Tris base is commercially available from Fisher Scientific Co. (Hanover, IL) and Sigma-Aldrich (St. Louis, MO), for example.

Moreover, in some embodiments, the inventive composition includes a salt, such as sodium chloride. The sodium chloride, when present, is preferably at a concentration of about 0.03 M to about 0.15 M. While not wishing to be bound by any one theory, it is believed that pH and salt concentration can affect the density and strength of the polymerized basement membrane gel.

In one embodiment, the inventive composition includes an extracellular matrix, exogenous heparin and exogenous fibronectin, the composition having a basic pH. This composition may further include sodium chloride.

In a further embodiment, the composition includes an extracellular matrix and exogenous laminin, the composition having a basic pH. This composition may further include sodium chloride.

As defined herein, the "extracellular matrix" (of which the basement membrane is a specific type), is any material produced by cells and secreted into the surrounding medium. It is noted that the term "extracellular matrix" may be used interchangeably herein with the term "basement membrane". The extracellular matrix can be secreted by cells to form an interstitial basement membrane that forms the framework to which cells are attached. The basement membrane separates cells from mesenchymal connective tissue and provides spatial orientation and the stability required for the growth and differentiation of cells. Extracellular matrix molecules or basement membranes have also been implicated in the sequestration, storage and presentation of growth factors to tissues.

The basement membrane includes various components. However, its major component is laminin, followed by collagen IV, heparan sulfate proteoglycans, entactin and nidogen. These components polymerize in constant proportions when redissolved and allowed to reconstitute.

Extracellular matrix molecules or basement membranes are known in the art and are commercially available. For example, an extracellular matrix from EHS mouse sarcoma tumor is available as Matrigel^{®} (Becton Dickinson Corp., Medford, MA), ECM gel (Sigma-Aldrich Co., St. Louis, MO), and Cultrex^{™} (Trevigen, Gaithersburg, MD).

The term "extracellular matrix" is art recognized. Its components include one or more of the following proteins: fibronectin, laminin, vitronectin, tenascin, entactin, thrombospondin, elastin, gelatin, collagen, fibrillin, merosin, anchorin, chondronectin, link protein, bone sialoprotein, osteocalcin, osteopontin, epinectin, hyaluronectin, undulin, epiligrin, and kalinin. Other extracellular matrix molecules are described in Kleinman et al., J. Biometer. Sci. Polymer Edn., 5: 1-11, (1993). It is intended that the term "extracellular matrix" encompass a presently unknown extracellular matrix that may be discovered in the future, since its characterization as an extracellular matrix will be readily determinable by persons skilled in the art.

As described above, the compositions of the present invention include an extracellular matrix component. In one desired embodiment, the extracellular matrix is provided as a basement membrane protein extract, such as that disclosed in U.S. Patent No. 4,829,000.
The protein extract is capable of polymerizing into a rigid stable gel (i.e., a 3-dimensional matrix) on heating. In particular, when incubated at temperatures from about 15-40°C (preferably from about 22-37°C) for a sufficient time, the extracellular matrix proteins polymerize. The proteins can remain in soluble form by maintaining a temperature of the composition lower than this range. Moreover, sodium chloride, or other salts, can be added to the composition to keep the proteins in soluble form and/or to affect the density and strength of the polymerized basement membrane gel. Preferably, the extracellular matrix component is maintained at a concentration of about 10mg/ml of the total composition.

In some embodiments, the composition includes fibronectin, which is exogenously added to the extracellular matrix component. Preferably, the exogenous fibronectin is human fibronectin. The exogenous fibronectin is present at concentrations of about 5 mg/ml to about 20 mg/ml of the total composition, preferably at concentrations of about 8 mg/ml to 12 mg/ml of the total composition. It is known that fibronectin binds to collagen IV, and possibly other components of the extracellular matrix. Therefore, while not wishing to be bound by any one theory, it is likely that fibronectin is introduced into the matrix, thereby altering its structure. Human fibronectin is commercially available from Sigma-Aldrich (St. Louis, MO), for example.

Moreover, in some embodiments, exogenous heparin is present in the inventive compositions. The exogenous heparin is maintained at a concentration of about 200mg/ml to about 800 mg/ml of the total composition, preferably at a concentration of about 300 mg/ml to about 600 mg/ml of the total composition. Heparin is known to bind to various growth factors, like VEGF and bFGF. Moreover, heparin is known to bind to collagen IV and to laminin. Therefore, while not wishing to be bound by any one theory, it is likely that heparin acts both by perturbing the structure of the extracellular matrix and by sequestering or blocking accessibility of growth factors in the composition to cells. Commercial sources of heparin include Calbiochem (La Jolla, CA) and Sigma-Aldrich (St. Louis, MO).

Furthermore, in some embodiments, the compositions of the present invention include exogenous laminin. The exogenous laminin is present at a concentration of about 1 mg/ml to about 10 mg/ml of the total composition, preferably at a concentration of about 2 mg/ml to about 4 mg/ml of the total composition. Laminin is known to bind to collagen IV. Moreover, laminin is known to contain binding sites for heparin. Therefore, while not wishing to be bound by any one theory, it is likely that laminin perturbs the structure of the extracellular matrix component. In desired embodiments, the laminin is human laminin. Human laminin is available from Sigma-Aldrich (St. Louis, MO), for example.

In addition to the components described above, the compositions of the present invention can also include various other components, which can affect the accessibility of the growth factors in the matrix to cells and/or which affect the structure of the matrix. These components include, but are not limited to, salts, diluents, heparan sulfate proteoglycans and/or neutralization antibodies. Additionally, suitable components of the composition can include those known in the art which do one or more of the following: affect the network density and strength when the proteins in the composition polymerize at temperatures of about 22°C to about 37°C, sequester or block accessibility of growth factors in the matrix to cells, alter the crosslink states of the polymerized matrix, or assist in cell adhesion.

The diluents can include any cell culture medium which provides a condition that is compatible to cell culture. Diluents appropriate for growth and differentiation of epithelial cells can include, but are not limited to Dulbecco's Modified Eagle Medium (DMEM), MEM, M-199 and RPMI. Supplements, as are known in the art, may be added to the culture medium and include serum (e.g., FBS or calf serum), serum-containing supplements (NU-SERUM), and serum-free supplements (MITO+). A preferred cell culture medium for intestinal epithelial cells is DMEM supplemented with MITO+ Serum Extender (Collaborative Biomedical Products, Bedford, Mass.) to provide a fully defined, serum-free cell culture environment. Moreover, many classical media have been used to culture epithelial hepatocytes. Some examples of these are: Liebovitz L-15, DMEM/F-12, RPMI 1640, Waymouth's MB 752/1 and Williams Medium E. Hepatocytes can also be cultured in a serum-free medium named Hepatocyte Medium, available from Sigma-Aldrich (St. Louis, MO).

Diluents appropriate for growing endothelial cells include, but are not limited to, DMEM, HUVEC medium and EGM2-MV medium. For example, Human Umbilical Vein Endothelial Cells (HUVEC) can be cultured in HUVEC medium (Sigma-Aldrich, St. Louis, MO). Moreover, human umbilical cord endothelial cells (Cascade Biologics, Inc., Portland, OR) can be cultured in EGM2-MV medium (Clonetics Corp., San Diego, CA., a division of Biowhittaker).

The neutralization antibodies which are suitable include those compounds which affect the growth factor inherent in the extracellular matrix by titrating out the growth factor or blocking their effectiveness. Suitable neutralization antibodies may include, but are not limited to, antibodies against one of the following: TGF-*beta*, EGF, VEGF, PDGF, bFGF and IGF-1.

A wide variety of other materials, including bioactive proteins, may be copolymerized with the extracellular matrix composition of the present invention. These include, but are not limited to, cells, antibodies, enzymes, receptors, growth factors, additional components of the extracellular matrix, cytokines, hormones and drugs. Polymerization of the extracellular matrix proteins can bind such materials. Moreover, the extracellular matrix proteins can copolymerize with the materials. These biologically active materials, if present, can be readily available to the cultured cells to moderate or regulate their properties or behavior.

The present invention provides a method of preparing an extracellular matrix composition, such as a reconstituted basement membrane composition. The inventive compositions are useful for coating a surface, such as a surface of a cell culture substrate. The method of preparation includes providing an extracellular matrix; and combining the provided extracellular matrix with at least one exogenous component selected from heparin, fibronectin and laminin. The method may further include adjusting the pH of the composition to a basic pH. Moreover, the method may included the step of adding a salt, such as sodium chloride, to the composition. As described above, it is believed that pH and salt concentration can affect the density and strength of the polymerized basement membrane gel.

The method of preparing the inventive compositions is generally performed as follows. Initially, a frozen basement membrane protein extract, such as Growth Factor Reduced Matrigel® (Becton Dickinson Corp., Medford, MA) is thawed on ice. Once the basement composition is thawed, at least one exogenous component selected from fibronectin, heparin and lamin is added. The pH of the composition can be adjusted to a basic pH. The basement membrane is desirably maintained at a concentration of about 10 mg/ml of the final product. In some embodiments, after the basement membrane composition is thawed, fibronectin and heparin are added. In other embodiments, after the basement membrane composition is thawed, laminin is added. The composition is maintained at a temperature of about 4°C for coating.

As described above, in some embodiments, the composition includes an extracellular matrix component, exogenous fibronectin and exogenous heparin. The composition may have a basic pH. In some embodiments, once the extracellular matrix component (e.g., a basement membrane protein extract) is thawed, fibronectin is added. The composition containing the exogenous fibronectin can then be dialyzed against a suitable cold dialysis buffer to adjust to a basic pH. For example, the composition can be dialyzed against the following dialysis buffer: DMEM (without NaHCO₃) containing an additional 40 mM NaCl/10mM Tris plus 50 µg/ml gentamycin, adjusted to pH 8.0. In particular, the composition containing the fibronectin may be placed in a dialysis bag and dialyzed aseptically for about 18-20 hours. The contents are then removed and heparin is added. It is noted that, alternatively, heparin can be added prior to dialysis. The concentration of the extracellular matrix component in the final product is desirably about 10 mg/ml, that of exogenous fibronectin is desirably about 5 mg/ml to about 20 mg/ml, and that of exogenous heparin is desirably about 200 mg/ml to about 800 mg/ml. The composition is maintained at a temperature of about 4°C for coating.

In other embodiments, the inventive composition includes an extracellular matrix component and exogenous laminin. The composition may have a basic pH. Once the extracellular matrix component is thawed as described above, laminin is added. The laminin may be added before or after the dialysis step. For example, in some embodiments, an extracellular matrix component, such as Growth Factor Reduced Matrigel® (Becton Dickinson Corp., Medford, MA), is first dialyzed against the following dialysis buffer: DMEM (without NaHCO₃) containing an additional 40 mM NaCl/10mM Tris plus 50 µg/ml gentamycin, adjusted to pH 8.0. After 18-20 hours of aseptic dialysis, laminin may then be added, such that the concentration of the exogenous laminin in the final product is about 1 mg/ml to about 10 mg/ml. The composition is maintained at a temperature of about 4°C for coating.

The present invention further provides a cell culturing system including: a substrate; and a coating thereon of a coating composition. The coating composition includes an extracellular matrix; and at least one exogenous component selected from heparin, fibronectin and laminin. In some embodiments, the extracellular matrix component to which the exogenous components are added is a basement membrane component, such that the coating composition is a modified reconstituted basement membrane composition. The coating composition may have a basic pH, Preferably, the pH is about 8.0.

Substrates for conventional cell culture research include plastic, glass, and micro porous filters (e.g., cellulosic, nylon, glass fiber, polyester, and polycarbonate). Substrates for bio-reactors used in batch or continuous cell culture or in genetic engineering include hollow fiber tubes or micro carrier beads. Substrates for vascular or bone grafts include materials such as polytetrafluoroethylene (Teflon®), ceramics and related polymeric materials.

In some embodiments, the substrate is selected from the following: cellulose membranes, porous polycarbonate, porous polytetrafluoroethylene, nylon membranes, glass filters, porous polyethyleneterephthalate, polymethylpentane, polypropylene, polyethylene and combinations thereof.

Preferred substrate configurations contemplated by the present invention include multiwell plates (such as 24-well and 96-well plates), dishes (such as petri dishes), culture flasks, etc.

The present invention provides a method of preparing a cell-culturing system. The method includes providing an extracellular matrix composition including an extracellular matrix; and at least one exogenous component selected from heparin, fibronectin and laminin; and applying the composition to a substrate. The method also includes incubating the applied composition to allow polymerization thereof. A suitable substrate for coating may be selected from those described above, for example. The composition applied to the substrate may have a basic pH, which is desirably about 7.8 to about 8.5. For example, in some embodiments, the coating composition includes a Tris buffer at a concentration sufficient to buffer in the basic pH range. Moreover, in some embodiments, the applied composition may include sodium chloride, desirably at a concentration of about 0.03 M to about 0.15 M.

The coating procedure is generally performed as follows. Approximately 0.5 to 2.0 ml of the coating composition (desirably at about 4°C) is applied to a well in a 6-well multiwell plate; about 0.25 to 1.0 ml is applied to a well in a 24-well multiwell plate; and about 50 µl to 100 µl is applied to a well in a 96-well plate. Furthermore, about 0.5 to 2.0 ml is applied to a 35 mm dish, 0.5 to 4.0 ml is applied to a 60 mm dish and 2.0 to 12.0 ml is applied to 100 mm dish. If desired, coated substrates can be frozen and stored at -20°C before use, and then thawed prior to polymerizing the components of the coating composition. In embodiments where the substrate configuration is that of a multiwell plate, a mat cover may be used to seal each plate before putting on the lid to prevent the surface from over drying. Commercially available mat covers include, for example, to those supplied by SUN-SRI, Wilmington, N.C.

After application, the inventive composition is incubated to permit adsorption of the cell adhesion substance(s) in the composition to the substrate surface and to permit polymerization of the proteins in the composition. In particular, coated substrates are desirably incubated at temperatures from about 22°C to about 37°C, and for a period of time of about 30 minutes to about 4 hours to effect polymerization of the components of the composition. If desired, the coating solution may be allowed to evaporate by drying at a temperature in this range and desirably at a relative humidity of 40-60%, and then rehydrated with cell culture medium or another sterile medium (e.g., sterile water) for about 2 hours at room temperature. The rehydration solution can then be removed just prior to assay setup. The final coated substrate is useful for assessing potential effects of stimulators, as well as inhibitors in that it produces a low background of cell tube formation or cell differentiation.

The compositions and cell culturing systems of the present invention can be used in various applications, including those known in the art for extracellular matrix and assay systems. These include *in vitro,* as well as *in vivo* applications. For example, one of the major *in vitro* applications includes the use of the inventive compositions in endothelial cell tube formation assays in response to exogenously added stimulators and/or inhibitors. Endothelial cell tube formation has been used as a model of endothelial cell differentiation, as well as the final step of the angiogenic cascade. Previously, background cell tube formation was too high in assays employing prior extracellular matrix compositions. This limited the application of the prior compositions to studies involving potential inhibitors of cell differentiation and/or angiogenesis. In contrast, the extracellular matrix compositions of the present invention are useful for stimulation studies, as well as inhibition studies. For example, with the present compositions, in the absence of an exogenous stimulator, background cell tube formation is insignificant. Thus, endothelial cell tube formation, and tube formation with other cell types, can be monitored in response to exogenously added stimulators, such as bFGF and sphinosine-1-phosphate. Additionally, cell differentiation of mammary ephelial cell acini formation, or neurite outgrowth can be studied using the inventive compositions. With respect *to in vivo* uses, the compositions of the present invention can be mixed with angiogenesis stimulators, inhibitors and/or tumor cells in a plug assay, which can be used to induce (e.g., with stimulators) or suppress (e.g., with inhibitors) blood vessel growth *in vivo.*

The assay system of the present invention can be used to test various inhibitors or stimulators to determine their effectiveness in a cell study. Stimulators can include growth factors which are known in the art. For example, these can include one or more of platelet derived growth factors (PDGF), e.g., PDGF AA, PDGF BB; insulin-like growth factors (IGF), e.g., IGF-I, IGF-II; fibroblast growth factors (FGF), e.g., acidic FGF, basic FGF, β-endothelial cell growth factor, FGF 4, FGF 5, FGF 6, FGF 7, FGF 8, and FGF 9; transforming growth factors (TGF), e.g., TGF-P1, TGF β1.2, TGF-β2, TGF-β 3, TGF-β 5; bone morphogenic proteins (BMP), e.g., BMP 1, BMP 2, BMP 3, BMP 4; vascular endothelial growth factors (VEGF), e.g., VEGF, placenta growth factor; epidermal growth factors (EGF), e.g., EGF, amphiregulin, betacellulin, heparin binding EGF; interleukins, e.g., IL-1, IL-2, IL-3, IL-4, IL-5, IL-6, IL-7, IL-8, IL-9, IL-10, IL-11, IL-12, IL-13, IL-14; colony stimulating factors (CSF), e.g., CSF-G, CSF-GM, CSF-M; nerve growth factor (NGF); stem cell factor; hepatocyte growth factor, and ciliary neurotrophic factor. Additional growth factors are described in Sporn and Roberts, Peptide Growth Factors and Their Receptors I, Springer-Verlag, New York (1990). The term "growth factors" is intended to encompass presently unknown growth factors that may be discovered in the future, since their characterization as a growth factor will be readily determinable by persons skilled in the art.

Many growth factors are available commercially from vendors, such as Sigma Chemical Co., St. Louis, MO.; Collaborative Research, Los Altos, CA; Genzyme, Cambridge, MA; Boehringer, Germany; R&D Systems, Minneapolis, MN; Genetech, San Francisco, CA; and GIBCO, Grand Island, NY. The commercially available growth factors may be obtained in both natural and recombinant forms.

The following examples are for illustrative purposes and are not intended to, in any way, limit the embodiments and uses of the present invention.

### EXAMPLES

### Example 1- Preparation of a Modified Extracellular Matrix Composition

A modified reconstituted basement membrane composition of the present invention was prepared as follows. Frozen Growth Factor Reduced Matrigel® (Becton Dickinson Corp., Medford, MA) was thawed on ice. After thawing, human fibronectin was added such that the concentration in the final product was about 8 mg/ml to about 12 mg/ml. The composition containing the exogenous fibronectin was dialyzed against the following dialysis buffer: DMEM (without NaHCO₃) containing an additional 40 mM NaCl/10mM Tris plus 50 µg/ml gentamycin, adjusted to pH 8.0. In particular, the composition containing the fibronectin was placed in a dialysis bag and dialyzed aseptically for about 18-20 hours. The contents were then removed and heparin was added. The concentration of the extracellular matrix component in the final product was about 10 mg/ml, and that of the exogenous heparin was about 300 mg/ml to about 600 mg/ml. The composition was maintained at a temperature of about 4°C prior to coating cell culture substrates.

### Example 2-General Coating Procedure

Multiwell plates were prepared as follows. Approximately 50µl of a solution (at 4°C) of either the modified reconstituted basement membrane composition described in Example 1 or standard Growth Factor Reduced Matrigel® (Becton Dickinson Corp., Medford, MA) was added to wells of BD Falcon Black/clear 95-well micro plates. A mat cover was used to seal each plate before putting on the lid. The plates were frozen and stored at -20°C before use.

### Example 3-General Procedure For Endothelial Cell Tube Formation Assay

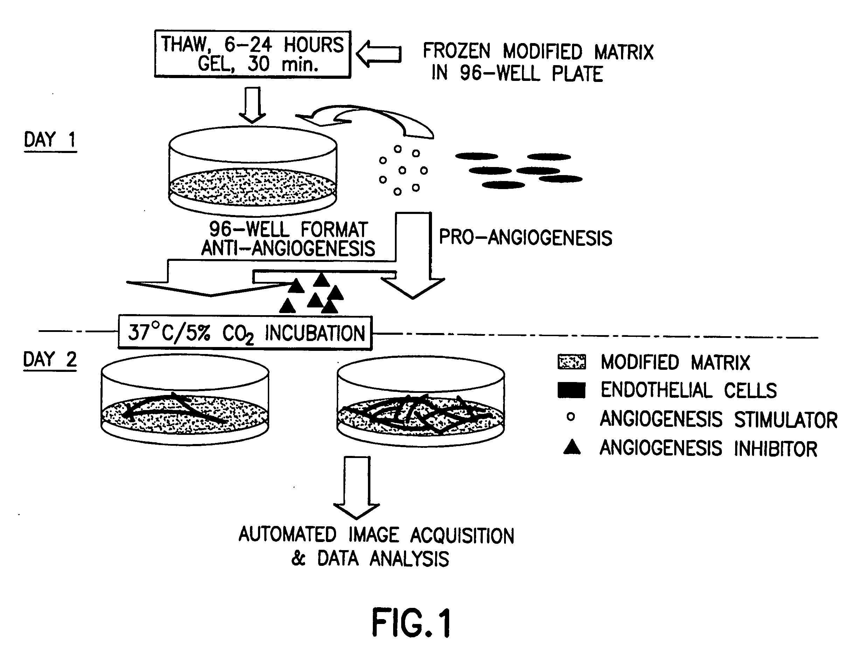

With reference to Figure 1, angiogenesis stimulators can be evaluated for their ability to enhance HUVEC tube formation using the compositions and cell culturing systems of the present invention. In particular, Figure 1 shows stimulators being evaluated on the coated surface of wells of a 96-well plate. The bottom surface of the wells are shown as coated with the modified matrix composition of the present invention. On day 1, a potential angiogenesis stimulator is added to the coated surface, along with endothelial cells in cell medium. As shown in Figure 1, the compositions and cell culturing systems of the present invention can also be used in inhibitor studies. In inhibitor studies, the potential inhibitor is included in a well, in addition to at least one known stimulator and the endothelial cells in cell medium. After an appropriate incubation period, typically 24 hours at 37°C/5% CO2 (day 2), images of the wells are acquired, and tube length is quantified as a measure of cell tube formation.

### Example 4- Stimulator Studies

Known stimulators were chosen to evaluate the performance of an inventive basement membrane composition vs. a prior basement membrane composition. The chosen stimulators were bFGF and spinosine-1-phosphate (S1P). Coated plates prepared as described above in Example 2 were thawed at about 4°C for a period of about 4 to 5 hours. The components of the composition were then allowed to polymerize at 37°C, 5% CO₂ for approximately 30 minutes immediately before assay setup. For stimulator studies, HUVEC (P3-P5) were harvested at about 70% cell confluence. About 50µl of cells at 4x10⁵/ml in 0.1% FBS/EBM-2 medium supplemented with or without the angiogenesis stimulators were seeded onto 96-well plates containing the pre-solidified (i.e., polymerized) modified reconstituted basement membrane composition (inventive) or a standard Matrigel® matrix (BD Growth Factor Reduced Matrigel® Matrix). The assembled assays were allowed to incubate in a 37°C incubator with 5% CO₂ for approximately 16 to 18 hours. Compounds were dissolved in DMF or water according to manufacturer's instructions.

At the end of each assay, assay medium was removed and plates were washed once with HBSS. 50µl Calcein AM at 8µg/ml in HBSS was added to each well and plates were incubated in a 37°C/5% CO₂ incubator for 35 minutes followed by washing once in HBSS. Images were acquired with a 2X objective lens on an automated Gen-1 Cell-based Screening System (Universal Imaging Corporation™). Total tube length of each well was measured using a revised (from previous) MetaMorph® Journal. Excel data was imported and analyzed using BD Gentest MPM/ADMET Software Program from BD Biosciences Discovery Labware.

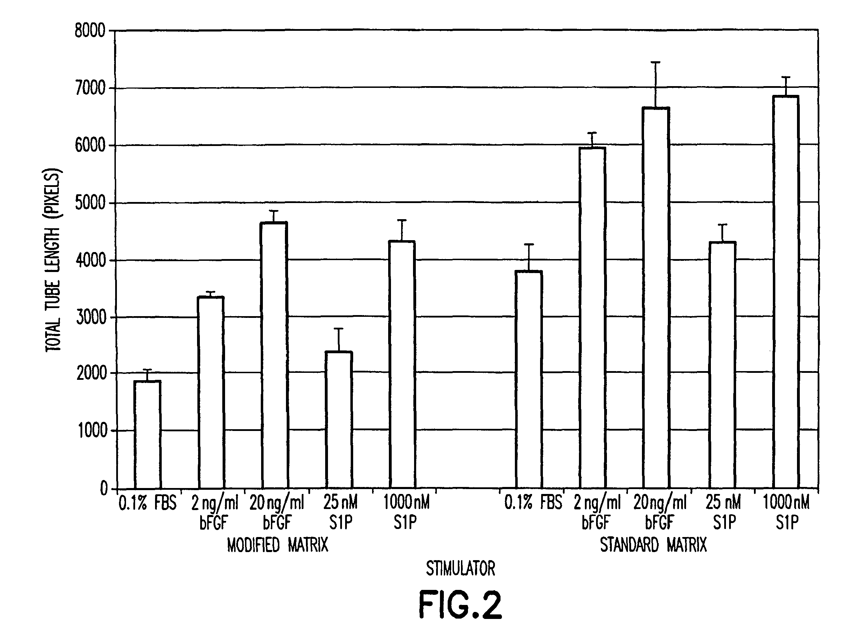

As shown in Figure 2, the tube length of HUVEC (a measure of cell tube formation) was compared using the different matrices in the presence of the stimulators, bFGF and S1P. The results show that the inventive composition (modified matrix) increases signal dynamic range over a standard basement membrane composition (standard matrix). The stimulators, bFGF and S1P, were incubated with HUVEC at the indicated concentrations on the matrices. Each bar represents the mean +/- standard Deviation (n=4). Figure 2 shows that the signal dynamic range was increased overall using the modified matrix of the present invention verses the standard matrix.

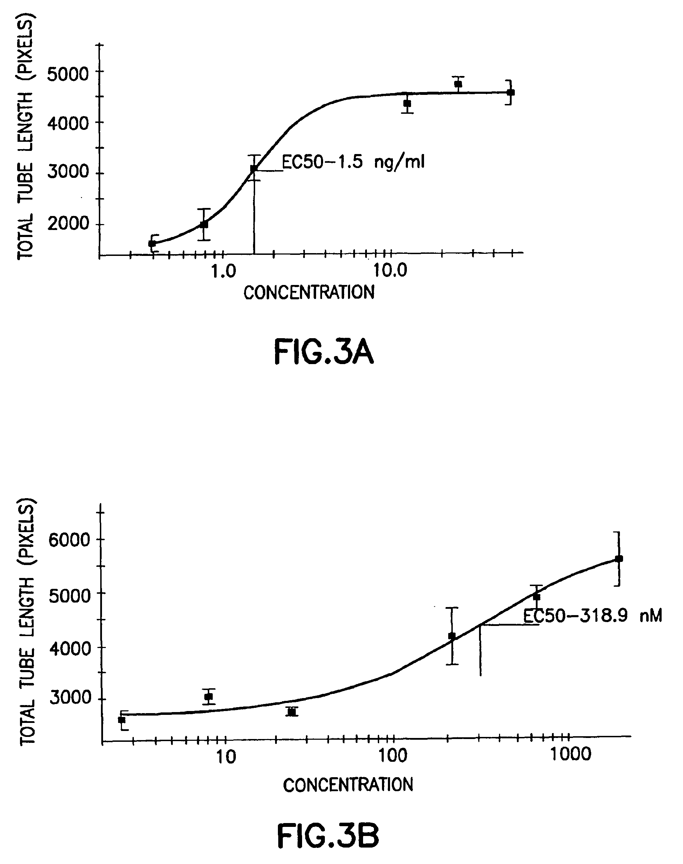

As shown in Figures 3A and 3B, the assay system allowed applicants to determine the EC₅₀ values of bFGF (Figure 3A) and S1P (Figure 3B) in inducing HUVEC tube formation. The dose response of the stimulators in inducing tube formation was obtained on the modified matrix (inventive) at the indicated concentrations. The stimulators, at indicted concentrations, were added to each well. Each data point represents the mean +/- Standard Deviation (n=4). The results show a dose-dependent stimulation of capillary-like structure formation by bFGF (Figure 3A) and S1P (Figure 3B). The inventive cell culturing system allowed applicants to determine an EC₅₀ value for bFGF of 1.5 ng/ml, and an EC₅₀ value for S1P of 318.9 nM.

### Example 5-Inhibitor Studies

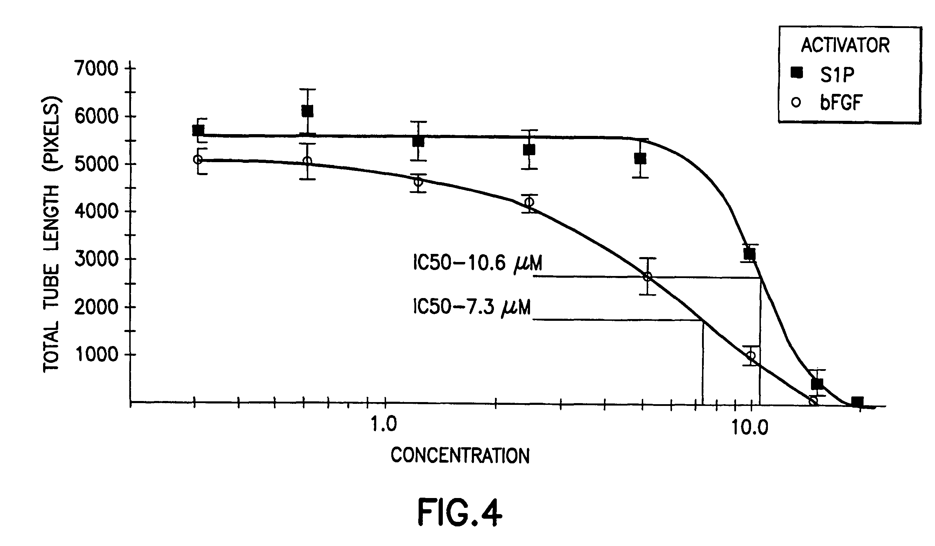

Inhibitor studies were performed as generally described in Example 4, except that for these studies, an inhibitor was included in the assay, in addition to a known stimulator. Data was obtained on the modified matrix (inventive). As shown in Figure 4, the total tube length induced by stimulators, bFGF or S1P, was measured in the presence of various concentrations of an inhibitor (Suramin). Stimulators, bFGF or S1P, were added to HUVECs at 20 ng/ml and 1µM, respectively. Suramin of indicated concentrations was added to HUVEC in bFGF or S1P prior to adding the mixtures to each well. Each data point represents the mean +/- standard deviation (n=4). Figure 4 shows that Suramin inhibited HUVEC tube formation induced by bFGF or S1P activation pathways with distinct IC₅₀ values. In particular, the IC₅₀ value of Suramin inhibition of tube formation stimulated by bFGF was 7.3 µM, and the IC₅₀ value of Suramin inhibition of tube formation stimulated by S1P was 10.6 µM.

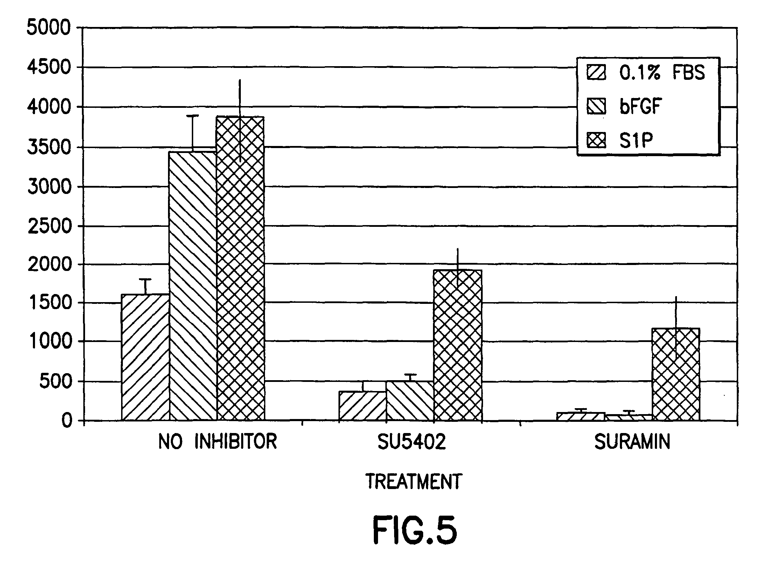

With reference now to Figure 5, the effects of inhibitors (SU5402 and Suramin) on HUVEC tube formation induced by stimulators( bFGF and S1P) is shown. Data was obtained on the modified matrix (inventive). Stimulators, bFGF or S1P, were added to HUVECs in EBM-2 containing 0.1% FBS at a final concentration of 20 ng/ml and 1µM, respectively. Suramin at 15µM (in medium) or SU5402 at 25µM (in 0.1% DMF) were added to HUVECs in 0.1% FBS, or bFGF, or S1P, prior to adding the mixtures to the wells. As shown in Figure 5, SU5402 (an FGFR1 tyrosine kinase inhibitor) partially inhibited S1P induced tube formation, raising the possibility that induction of bFGF could be one mechanism of S1P action. Moreover, SU5402 completely inhibited tube formation stimulated by bFGF. Furthermore, the graph shows that Suramin inhibited both S1P and bFGF induced tube formation. Each bar represents the mean +/standard deviation (n=4).

Modified reconstituted basement membrane of the present invention increased dynamic range and allowed assessment of the effect of both stimulators and inhibitors. The assay system allowed one to determine EC₅₀ values of stimulators in inducing HUVEC tube formation and IC₅₀ values of inhibitors of tube formation stimulated by the stimulatory agents. This assay system offers an opportunity to study action pathways of angiogenesis modulators in endothelial cell tube formation.

## Claims

1. An extracellular matrix composition for coating a surface wherein the pH of the composition is between 7.8 to 8.5 and which composition comprises
- an extracellular matrix; and
- at least one exogenous component selected from the group consisting of fibronectin present at concentrations of 5 mg/ml to 20 mg/ml of the composition, heparin present in concentrations of 200 mg/ml to 800 mg/ml of the composition, and laminin present in concentrations of 1 mg/ml to 10 mg/ml of the composition.

2. The composition of claim 1, wherein the extracellular matrix comprises a basement membrane protein extract.

3. The composition of claim 1, further comprising a Tris buffer at a concentration sufficient to buffer in the basic pH range.

4. The composition of claim 3, wherein the Tris buffer is present at a concentration of 0.01 M to 0.05 M of the composition.

5. The composition of claim 1, further comprising sodium chloride.

6. The composition of claim 5, wherein the sodium chloride is present at a concentration of 0.03 M to 0.15 M of the composition.

7. The composition of claim 1, wherein the extracellular matrix is present at a concentration of 10 mg/ml of the composition.

8. The composition of claim 1 comprising an extracellular matrix; and exogenous fibronectin and heparin, wherein the extracellular matrix has a concentration of 10 mg/ml, the fibronectin is present in concentrations of 5 mg/ml to 20 mg/ml and heparin is present in concentrations of 200 mg/ml to 800 mg/ml.

9. A cell culturing system comprising: a substrate; and a coating thereon of the extracellular matrix composition as defined in any of claims 1 to 8.

10. The system of claim 9, wherein the substrate is selected from the group consisting of cellulose membranes, porous polycarbonate, porous polytetrafluoroethylene, nylon membranes, glass filters, porous polyethyleneterephthalate, polymethylpentane, polypropylene, polyethylene and combinations thereof.

11. A method of preparing an extracellular matrix composition for coating a surface comprising:
(a) providing an extracellular matrix; and
(b) combining the provided extracellular matrix with at least one exogenous component selected from the group consisting of
fibronectin present at concentrations of 5 mg/ml to 20 mg/ml of the composition, heparin present in concentrations of 200 mg/ml to 800 mg/ml of the composition, and laminin present in concentrations of 1 mg/ml to 10 mg/ml of the composition; and
(c) adjusting the pH of the composition to a pH of between 7.8 and 8.5.

12. The method of claim 11, further comprising adding sodium chloride to the composition.

13. A method of preparing a cell-culturing system comprising:
(a) providing the extracellular matrix composition as defined in any of claims 1 to 8;
(b) applying the composition to a substrate; and
(c) incubating the applied composition to allow polymerization thereof.

14. The method of claim 13, wherein the incubating is performed at a temperature of 22°C to 37° C.

15. The method of claim 13, wherein the composition further comprises a Tris buffer at a concentration sufficient to buffer in the basic pH range.

16. The method of claim 13, wherein the composition further comprises sodium chloride.

17. The method of claim 13, wherein the substrate is selected from the group consisting of cellulose membranes, porous polycarbonate, porous polytetrafluoroethylene, nylon membranes, glass filters, porous polyethyleneterephthalate, polymethylpentane, polypropylene, polyethylene and combinations thereof.

## Patentansprüche

1. Extrazelluläre-Matrix-Zusammensetzung zur Beschichtung einer Oberfläche, wobei der pH-Wert der Zusammensetzung zwischen 7,8 und 8,5 liegt und wobei die Zusammensetzung Folgendes umfasst:
- eine extrazelluläre Matrix; und
- wenigstens eine exogene Komponente, die aus der Gruppe ausgewählt ist, die aus
Fibronectin in Konzentrationen von 5 mg/ml bis 20 mg/ml der Zusammensetzung;
Heparin in Konzentrationen von 200 mg/ml bis 800 mg/ml der Zusammensetzung; und
Laminin in Konzentrationen von 1 mg/ml bis 10 mg/ml der Zusammensetzung
besteht.

2. Zusammensetzung gemäß Anspruch 1, wobei die extrazelluläre Matrix ein Basalmembran-Proteinextrakt umfasst.

3. Zusammensetzung gemäß Anspruch 1, die weiterhin einen Tris-Puffer in einer ausreichenden Konzentration, um im basischen pH-Bereich zu puffern, umfasst.

4. Zusammensetzung gemäß Anspruch 3, wobei der Tris-Puffer in einer Konzentration von 0,01 M bis 0,05 M der Zusammensetzung vorhanden ist.

5. Zusammensetzung gemäß Anspruch 1, die weiterhin Natriumchlorid umfasst.

6. Zusammensetzung gemäß Anspruch 5, wobei das Natriumchlorid in einer Konzentration von 0,03 M bis 0,15 M der Zusammensetzung vorhanden ist.

7. Zusammensetzung gemäß Anspruch 1, wobei die extrazelluläre Matrix in einer Konzentration von 10 mg/ml der Zusammensetzung vorhanden ist.

8. Zusammensetzung gemäß Anspruch 1, die eine extrazelluläre Matrix und exogenes Fibronectin und Heparin umfasst, wobei die extrazelluläre Matrix eine Konzentration von 10 mg/ml hat, das Fibronectin in Konzentrationen von 5 mg/ml bis 20 mg/ml vorhanden ist und Heparin in Konzentrationen von 200 mg/ml bis 800 mg/ml vorhanden ist.

9. Zellkultursystem, umfassend: ein Substrat und darauf eine Beschichtung mit der Extrazelluläre-Matrix-Zusammensetzung gemäß einem der Ansprüche 1 bis 8.

10. System gemäß Anspruch 9, wobei das Substrat aus der Gruppe ausgewählt ist, die aus Cellulosemembranen, porösem Polycarbonat, porösem Polytetrafluorethylen, Nylonmembranen, Glasfiltern, porösem Polyethylenterephthalat, Polymethylpentan, Polypropylen, Polyethylen und Kombinationen davon besteht.

11. Verfahren zur Herstellung einer Extrazelluläre-Matrix-Zusammensetzung zur Beschichtung einer Oberfläche, umfassend:
(a) Bereitstellen einer extrazellulären Matrix; und
(b) Kombinieren der bereitgestellten extrazellulären Matrix mit wenigstens einer exogenen Komponente, die aus der Gruppe ausgewählt ist, die aus
Fibronectin in Konzentrationen von 5 mg/ml bis 20 mg/ml der Zusammensetzung;
Heparin in Konzentrationen von 200 mg/ml bis 800 mg/ml der Zusammensetzung; und
Laminin in Konzentrationen von 1 mg/ml bis 10 mg/ml der Zusammensetzung
besteht; und
(c) Einstellen des pH-Werts der Zusammensetzung auf einen pH zwischen 7,8 und 8,5.

12. Verfahren gemäß Anspruch 11, das weiterhin das Hinzufügen von Natriumchlorid zu der Zusammensetzung umfasst.

13. Verfahren zur Herstellung eines Zellkultursystems, umfassend:
(a) Bereitstellen der Extrazelluläre-Matrix-Zusammensetzung gemäß einem der Ansprüche 1 bis 8;
(b) Auftragen der Zusammensetzung auf ein Substrat; und
(c) Inkubieren der aufgetragenen Zusammensetzung, so dass sie polymerisiert.

14. Verfahren gemäß Anspruch 13, wobei das Inkubieren bei einer Temperatur von 22 °C bis 37 °C durchgeführt wird.

15. Verfahren gemäß Anspruch 13, wobei die Zusammensetzung weiterhin einen Tris-Puffer in einer ausreichenden Konzentration, um im basischen pH-Bereich zu puffern, umfasst.

16. Verfahren gemäß Anspruch 13, wobei die Zusammensetzung weiterhin Natriumchlorid umfasst.

17. Verfahren gemäß Anspruch 13, wobei das Substrat aus der Gruppe ausgewählt ist, die aus Cellulosemembranen, porösem Polycarbonat, porösem Polytetrafluorethylen, Nylonmembranen, Glasfiltern, porösem Polyethylenterephthalat, Polymethylpentan, Polypropylen, Polyethylen und Kombinationen davon besteht.

## Revendications

1. Composition de matrice extracellulaire pour enduire une surface, dans laquelle le pH de la composition est entre 7,8 et 8,5, la composition comprenant:
- une matrice extracellulaire; et
- au moins un composant exogène choisi dans le groupe consistant en
fibronectine présente en concentrations de 5 mg/ml à 20 mg/ml de la composition;
héparine présente en concentrations de 200 mg/ml à 800 mg/ml de la composition; et
laminine présente en concentrations de 1 mg/ml à 10 mg/ml de la composition.

2. Composition selon la revendication 1, dans laquelle la matrice extracellulaire comprend un extrait de protéines de la membrane basale.

3. Composition selon la revendication 1, comprenant en outre un tampon Tris en une concentration suffisante pour tamponner dans une gamme de pH basique.

4. Composition selon la revendication 3, dans laquelle le tampon Tris est présent en une concentration de 0,01 M à 0,05 M de la composition.

5. Composition selon la revendication 1, comprenant en outre du chlorure de sodium.

6. Composition selon la revendication 5, dans laquelle le chlorure de sodium est présent en une concentration de 0,03 M à 0,15 M de la composition.

7. Composition selon la revendication 1, dans laquelle la matrice extracellulaire est présente en une concentration de 10 mg/ml de la composition.

8. Composition selon la revendication 1, comprenant une matrice extracellulaire et de la fibronectine et de l'héparine exogènes, dans laquelle la matrice extracellulaire a une concentration de 10 mg/ml, la fibronectine est présente en concentrations de 5 mg/ml à 20 mg/ml et l'héparine est présente en concentrations de 200 mg/ml à 800 mg/ml.

9. Système de culture cellulaire, comprenant: un substrat et par dessus un revêtement de la composition de matrice extracellulaire telle que définie dans l'une quelconque des revendications 1 à 8.

10. Système selon la revendication 9, dans lequel le substrat est choisi dans le groupe consistant en membranes cellulosiques, polycarbonate poreux, polytétrafiluoroéthylène poreux, membranes de nylon, filtres en verre, polyéthylène téréphtalate poreux, polyméthylpentane, polypropylène, polyéthylène et des combinaisons de ceux-ci.

11. Procédé pour préparer une composition de matrice extracellulaire pour enduire une surface, comprenant les étapes consistant à:
(a) procurer une matrice extracellulaire; et
(b) combiner la matrice extracellulaire procurée avec au moins un composant exogène choisi dans le groupe consistant en
fibronectine présente en concentrations de 5 mg/ml à 20 mg/ml de la composition;
héparine présente en concentrations de 200 mg/ml à 800 mg/ml de la composition; et
laminine présente en concentrations de 1 mg/ml à 10 mg/ml de la composition;
(c) ajuster le pH de la composition à un pH compris entre 7,8 et 8,5.

12. Procédé selon la revendication 11, comprenant en outre l'étape consistant à ajouter du chlorure de sodium à la composition.

13. Procédé pour préparer un système de culture cellulaire, comprenant les étapes consistant à:
(a) procurer la composition de matrice extracellulaire telle que définie dans l'une quelconque des revendications 1 à 8;
(b) appliquer la composition sur un substrat; et
(c) incuber la composition appliquée pour la faire polymériser.

14. Procédé selon la revendication 13, dans lequel l'étape d'incubation est effectuée à une température de 22 °C à 37 °C.

15. Procédé selon la revendication 13, dans lequel la composition comprend en outre un tampon Tris en une concentration suffisante pour tamponner dans une gamme de pH basique.

16. Procédé selon la revendication 13, dans lequel la composition comprend en outre du chlorure de sodium.

17. Procédé selon la revendication 13, dans lequel le substrat est choisi dans le groupe consistant en membranes cellulosiques, polycarbonate poreux, polytétrafluoroéthylène poreux, membranes de nylon, filtres en verre, polyéthylène téréphtalate poreux, polyméthylpentane, polypropylène, polyéthylène et des combinaisons de ceux-ci.
